(19) **Europäisches Patentamt / European Patent Office / Office européen des brevets**

(11)  **EP 4 108 327 B1**

(12)  # EUROPEAN PATENT SPECIFICATION

(45) Date of publication and mention
of the grant of the patent:
**04.03.2026  Bulletin 2026/10**

(21) Application number: **21898345.0**

(22) Date of filing: **14.10.2021**

(51) International Patent Classification (IPC):
**B01J 37/04** (2006.01)      **B01J 37/08** (2006.01)
**B01J 37/00** (2006.01)      **B01J 37/06** (2006.01)
**B01J 27/18** (2006.01)      **C07C 51/377** (2006.01)
**C07C 57/04** (2006.01)

(52) Cooperative Patent Classification (CPC):
(C-Sets available)
**B01J 37/04; B01J 23/02; B01J 27/1806;
B01J 37/031; B01J 37/06; B01J 37/088;
C07C 51/377**                        (Cont.)

(86) International application number:
**PCT/KR2021/014200**

(87) International publication number:
**WO 2022/114519 (02.06.2022 Gazette 2022/22)**

(54) **METHOD FOR PRODUCING CATALYST FOR DEHYDROGENATION OF 3-HYDROXYPROPIONIC ACID, CATALYST FOR DEHYDROGENATION OF 3-HYDROXYPROPIONIC ACID, AND METHOD FOR PRODUCING ACRYLIC ACID USING SAME**

VERFAHREN ZUR HERSTELLUNG EINES KATALYSATORS ZUR DEHYDRIERUNG VON 3-HYDROXYPROPIONSÄURE, KATALYSATOR ZUR DEHYDRIERUNG VON 3-HYDROXYPROPIONSÄURE UND VERFAHREN ZUR HERSTELLUNG VON ACRYLSÄURE DAMIT

PROCÉDÉ DE PRODUCTION D'UN CATALYSEUR POUR LA DÉSHYDROGÉNATION D'ACIDE 3-HYDROXYPROPIONIQUE, CATALYSEUR POUR LA DÉSHYDROGÉNATION D'ACIDE 3-HYDROXYPROPIONIQUE, ET PROCÉDÉ DE PRODUCTION D'ACIDE ACRYLIQUE L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2020  KR 20200163037**

(43) Date of publication of application:
**28.12.2022  Bulletin 2022/52**

(73) Proprietor: **LG Chem, Ltd.
Seoul 07336 (KR)**

(72) Inventors:
• **KIM, Jaeyoung
Daejeon 34122 (KR)**

• **BANG, Jungup
Daejeon 34122 (KR)**
• **CHOI, Jae Soon
Daejeon 34122 (KR)**

(74) Representative: **Goddar, Heinz J.
Boehmert & Boehmert
Anwaltspartnerschaft mbB
Pettenkoferstrasse 22
80336 München (DE)**

(56) References cited:
**WO-A1-2013/155295      KR-A- 20120 025 888
KR-A- 20120 025 888      KR-A- 20120 096 125
KR-A- 20120 096 125      KR-A- 20130 132 850
KR-A- 20170 001 088**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 4 108 327 B1

(52) Cooperative Patent Classification (CPC): (Cont.)

C-Sets
**C07C 51/377, C07C 57/04**

**Description**

[Technical Field]

The present specification relates to a method for preparing a catalyst for dehydration reaction of 3-hydroxypropionic acid, the catalyst for dehydration reaction of 3-hydroxypropionic acid, and a method for producing acrylic acid using the same.

[Background Art]

3-Hydroxypropionic acid (3-HP) is a platform compound capable of being converted into various substances and may be obtained from glucose or glycerol through a biological conversion process.

3-hydroxypropionic acid (3-HP) may be converted to acrylic acid through a dehydration reaction, wherein the dehydration reaction includes: solid acid catalysts such as $SiO_2$, $TiO_2$, $Al_2O_3$, and zeolite; base catalysts such as ammonia, polyvinylpyrrolidine, metal hydroxide, and $Zr(OH)_4$; or metal catalysts such as $MgSO_4$, $Al_2(SO_4)_3$, $K_2SO_4$, $AlPO_4$, and $Zr(SO_4)_2$, and the yield of acrylic acid produced is a level of 60 to 90%.

<Prior Art Document>

(Patent Document) KR 10-2012-0025888 A

KR 20120025888 A discloses a method for preparing a catalyst, wherein an apatite cake is prepared by generally mixing an aqueous solution containing dissolved calcium salts and an aqueous solution containing dissolved phosphate, and a disodium calcium phosphate cake is prepared by generally mixing an aqueous solution containing dissolved phosphate with an aqueous solution containing dissolved calcium salts. Always, the aqueous solution containing dissolved calcium salts is added to the solution containing dissolved phosphate.

[Disclosure]

[Technical Problem]

The present disclosure relates to a method for preparing a catalyst for dehydration reaction of 3-hydroxypropionic acid, the catalyst for dehydration reaction of 3-hydroxypropionic acid, and a method for producing acrylic acid using the same.

[Technical Solution]

An embodiment of the present disclosure provides a method for preparing a catalyst for dehydration reaction of 3-hydroxypropionic acid, including hydroxyapatite (HAP) and calcium pyrophosphate, the method comprising the steps of:

preparing an apatite cake by dropping a first phosphate solution into a first calcium salt solution;
preparing a calcium pyrophosphate (CaPP) cake by dropping a second phosphate solution into a second calcium salt solution;
preparing a calcium phosphate cake by mixing the apatite cake and the calcium pyrophosphate cake; and
firing the calcium phosphate cake.

An embodiment of the present disclosure provides a catalyst for dehydration reaction of 3-hydroxypropionic acid, obtainable by the inventive method, in which the hydroxyapatite phase and the calcium pyrophosphate phase of the catalyst have a weight ratio of 10:90 to 80:20, as a catalyst for dehydration reaction of 3-hydroxypropionic acid, including a hydroxyapatite phase and a calcium pyrophosphate phase.

Furthermore, an embodiment of the present disclosure provides a method for producing acrylic acid, comprising the step of producing acrylic acid by performing dehydration reaction of 3-hydroxypropionic acid using the catalyst.

Further embodiments are disclosed in the dependent claims.

[Advantageous Effects]

The catalyst prepared by the method for preparing a catalyst for dehydration reaction of 3-hydroxypropionic acid according to an embodiment of the present disclosure has an effect of exhibiting a high acrylic acid yield when used in a process for producing acrylic acid by performing dehydration reaction of 3-hydroxypropionic acid.

[Best Mode for Carrying Out the Invention]

In the present specification, when a part 'includes' a certain component, it means that other components may be further included, rather than excluding other components, unless there is a particular opposite description.

The term 'phase' of a substance in the present specification means a state in which certain substances are gathered to form a system or group having uniform physical and chemical properties from a macroscopic point of view. The existence of such a phase can be confirmed through XRD analysis under specific voltage, current, scan speed and scan step conditions using Bruker's D4 endeavor X-ray diffractometer. That is, a peak occurring within a specific $2\theta$ (theta) range may be observed and confirmed by the result of XRD analysis.

Hereinafter, the present disclosure will be described in more detail.

An embodiment of the present disclosure provides a method for preparing a catalyst for dehydration reaction of 3-hydroxypropionic acid, including hydroxyapatite (HAP) and calcium pyrophosphate, the method comprising the steps of:

preparing an apatite cake by dropping a first phosphate solution into a first calcium salt solution;
preparing a calcium pyrophosphate (CaPP) cake by dropping a second phosphate solution into a second calcium salt solution;
preparing a calcium phosphate cake by mixing the apatite cake and the calcium pyrophosphate cake; and
firing the calcium phosphate cake.

When the catalyst has a single phase of hydroxyapatite (HAP) or calcium pyrophosphate, there has been a problem in that the yield of acrylic acid in the 3-hydroxypropionic acid dehydration reaction is lowered and the processability is deteriorated due to a short lifespan.

Meanwhile, a catalyst comprising hydroxyapatite and calcium pyrophosphate has advantages in that the yield of the dehydration reaction is improved, and the processability is improved due to a long lifespan. When the catalyst comprises hydroxyapatite and calcium pyrophosphate, it may be defined as having a mixed phase of hydroxyapatite and calcium pyrophosphate. The mixed phase means that hydroxyapatite and calcium pyrophosphate are simply mixed.

The apatite cake and the calcium pyrophosphate cake are prepared by reacting a phosphate solution and a calcium salt solution with each other, and the present disclosure has been completed by studying that the mixed phase is easily formed when preparing each cake by dropping the phosphate solution into the calcium salt solution as the mother liquid instead of dropping the calcium salt solution into the phosphate solution as a mother liquid as in the conventional art.

Specifically, a method for preparing a catalyst according to an embodiment of the present disclosure comprises the steps of: preparing an apatite cake by dropping a first phosphate solution into a calcium salt solution; and preparing a calcium pyrophosphate cake by dropping a second phosphate solution into the calcium salt solution.

In the present specification, dropping means dropping a solution in the form of drops, and the dropping amount may be adjusted by adjusting the dropping rate (unit: volume/time, e.g., mL/min) and the dropping time. A conventional method in the art may be used for the dropping.

In an embodiment of the present disclosure, the step of preparing the apatite cake by dropping the first phosphate solution into the first calcium salt solution comprises a step of dropping the first phosphate solution into a bath containing the first calcium salt solution, and the step of preparing the calcium pyrophosphate cake by dropping the second phosphate solution into the second calcium salt solution comprises a step of dropping the second phosphate solution into a bath containing the second calcium salt solution.

In an embodiment of the present disclosure, the step of preparing the apatite cake by dropping the first phosphate solution into the first calcium salt solution comprises a step of dropping the first phosphate solution into a bath containing the first calcium salt solution. Specifically, after preparing the bath containing the first calcium salt solution, the first phosphate solution may be slowly dropped into the bath.

In an embodiment of the present disclosure, the step of preparing the calcium pyrophosphate cake by dropping the phosphate solution into the second calcium salt solution comprises a step of dropping the second phosphate solution into the bath containing the second calcium salt solution. Specifically, after preparing the bath containing the second calcium salt solution, the second phosphate solution may be slowly dropped into the bath.

The dropping rate may be 0.1 mL/min or more and 10 mL/min or less, or 1 mL/min or more and 10 mL/min or less respectively. If it falls short of the above range, the reaction is slow to deteriorate processability, and if the rate is exceeded, a reaction may occur rapidly and precipitates may be generated so that it is preferable to adjust the reaction within the above range.

The step of preparing a calcium phosphate salt by dropping the phosphate solution into the calcium salt solution is different from a conventional method of preparing the calcium phosphate salt by dropping the calcium salt solution into the phosphate solution. When dropping the calcium salt solution into the phosphate solution as a mother liquid as in the conventional method, there has been a problem in that a sodium pyrophosphate ($Na_2CaP_2O$-) hydrate is produced and a calcium pyrophosphate phase is not well formed after heat treatment so that the mixed phase of hydroxyapatite (HAP) and calcium pyrophosphate is not formed well.

Meanwhile, as in an embodiment of the present disclosure, when preparing the calcium phosphate salt by dropping the phosphate solution into the calcium salt solution, the mixed phase of hydroxyapatite and calcium pyrophosphate may be easily formed due to the formation of a calcium pyrophosphate ($Ca_2P_2O_7$) hydrate.

Whether the mixed phase exists or not may be confirmed using XRD (X-ray diffraction) analysis of the prepared catalyst. At this time, XRD analysis may use Bruker's D4 endeavor X-ray diffractometer, and whether the mixed phase exists or not may be measured by a method of observing a peak occurring within a $2\Theta$ (theta) range of 10 to 60 degrees (°) under CuKa lamp, 40 kV voltage, 40 mA current, 0.8 deg/min scan speed, and 0.015 scan step conditions.

Specifically, it is confirmed that the mixed phase exists by confirming that the peak corresponding to the hydroxyapatite phase at $2\Theta$ (theta) angles of 25.9 degrees (°) and 31.7 degrees (°) and the peak corresponding to the calcium pyrophosphate (CaPP) phase at $2\Theta$ (theta) angles of 26.7 degrees (°) and 30.5 degrees (°) appear at the same time.

In an embodiment of the present specification, the apatite cake and the calcium pyrophosphate cake in the step of preparing the calcium phosphate cake by mixing the apatite cake and the calcium pyrophosphate cake may have a weight ratio of 10:90 to 80:20, 15:85 to 70:30, 18:82 to 55:45, or 20:80 to 50:50.

Further, in an embodiment of the present specification, the apatite cake may have a weight ratio smaller than that of the calcium pyrophosphate cake in the step of preparing the calcium phosphate cake by mixing the apatite cake and the calcium pyrophosphate cake. When the apatite cake has a weight ratio smaller than that of the calcium pyrophosphate cake in the step of preparing the calcium phosphate cake by mixing the apatite cake and the calcium pyrophosphate cake, the yield of acrylic acid may be further increased when producing acrylic acid using a catalyst for dehydration reaction of hydroxypropionic acid prepared using the calcium phosphate cake.

In an embodiment of the present specification, the first calcium salt solution and the second calcium salt solution may each have a calcium salt concentration of 0.1M or more and 5M or less.

In an embodiment of the present specification, the first calcium salt solution and the second calcium salt solution may each have a calcium salt concentration of 0.1M or more and 5M or less, preferably 0.3M or more and 2M or less, and more preferably 1M or more and 1.5M or less. When the above range is exceeded, there is a problem in that the particles are hardened during stirring due to gelation of calcium pyrophosphate, and when it falls short of the above range, there is a problem in that catalyst productivity is low.

In an embodiment of the present specification, the first phosphate solution and the second phosphate solution may each have a phosphate concentration of 0.1M or more and 5M or less.

In an embodiment of the present specification, the first phosphate solution and the second phosphate solution may each have a phosphate concentration of 0.1M or more and 5M or less, preferably 0.3M or more and 2M or less, and more preferably 0.5M or more and 1M or less. When the above range is exceeded, there is a problem in that the particles are hardened during stirring due to gelation of calcium pyrophosphate, and when it falls short of the above range, there is a problem in that catalyst productivity is low.

In an embodiment of the present specification, the dropping of the first phosphate solution and the dropping of the second phosphate solution may be performed for 25 seconds or more and 1 hour or less, and preferably 30 seconds or more and 1 hour or less.

In an embodiment of the present specification, the first phosphate may be one or more selected from the group consisting of $Li_3PO_4$, $Na_3PO_4$, and $K_3PO_4$, or hydrates thereof.

In an embodiment of the present specification, the second phosphate may be one or more selected from the group consisting of pyrophosphoric acid ($H_4P_2O_7$), $Li_4P_2O_7$, $Na_4P_2O_7$, and $K_4P_2O_7$, or hydrates thereof.

In an embodiment of the present specification, the first calcium salt solution and the second calcium salt solution may each contain one or more calcium salts selected from the group consisting of calcium chloride, calcium nitrate, calcium sulfate, and calcium acetate, or hydrates thereof.

In an embodiment of the present specification, the step of preparing the calcium phosphate cake by mixing the phosphate solution and the calcium salt solution may comprise a step of performing stirring at a temperature of 20 to 60 °C for 1 to 48 hours.

In an embodiment of the present specification, the step of preparing the calcium phosphate cake by mixing the apatite cake and the calcium pyrophosphate cake may comprise the steps of: preparing a calcium phosphate slurry by mixing the apatite cake and the calcium pyrophosphate cake; and filtering, washing, and drying the calcium phosphate slurry to prepare a calcium phosphate cake.

In an embodiment of the present specification, the step of preparing the calcium phosphate cake by mixing the apatite cake and the calcium pyrophosphate cake may further comprise a step of pulverizing the calcium phosphate cake.

The step of pulverizing the calcium phosphate cake may comprise a step of pulverizing the calcium phosphate cake into a powder having an average particle size (D50) of 5 to 100 $\mu$m. The average particle size (D50) means a particle size when the cumulative percentage in the average particle size distribution reaches 50%. For the average particle size (D50), Horiba's Laser Particle Size Analyzer LA-950 equipment may be used, a dispersion is prepared by diluting a solution containing the calcium phosphate powder with water, and it may be measured in a temperature range of 15 to 35 °C by injecting the dispersion into a Laser Particle Size Analyzer.

In an embodiment of the present specification, the step of filtering, washing, and drying the calcium phosphate slurry may be performed at 80 to 120 °C for 5 to 48 hours.

In an embodiment of the present specification, the method may further comprise a step of molding the calcium phosphate cake or powder into calcium phosphate pellets.

In an embodiment of the present specification, the step of firing the calcium phosphate cake may be performed at a temperature condition of 300 to 700 °C. The temperature condition may be adjusted to 400 to 600 °C, or 450 to 550 °C so that the firing can be sufficiently performed.

In an embodiment of the present specification, the step of firing the calcium phosphate cake may be performed for 1 to 24 hours.

An embodiment of the present disclosure provides a catalyst for dehydration reaction of 3-hydroxypropionic acid including a hydroxyapatite phase and a calcium pyrophosphate phase. The catalyst may be prepared by the above-described preparation method.

In an embodiment of the present specification, the hydroxyapatite phase and the calcium pyrophosphate phase of the catalyst for dehydration reaction may have a weight ratio of 10:90 to 80:20.

In an embodiment of the present specification, the hydroxyapatite phase and the calcium pyrophosphate phase of the catalyst for dehydration reaction may have a weight ratio of 10:90 to 80:20, 15:85 to 70:30, 18:82 to 55:45, or 20:80 to 50:50. That is, it is the same as the weight ratio of the apatite cake and the calcium pyrophosphate cake in the step of preparing the calcium phosphate cake by mixing the apatite cake and the calcium pyrophosphate cake.

Further, in an embodiment of the present specification, the hydroxyapatite phase of the catalyst for dehydration reaction may have a weight ratio smaller than that of the calcium pyrophosphate phase.

When the weight ratio of the hydroxyapatite phase of the catalyst for dehydration reaction is smaller than that of the calcium pyrophosphate phase, the yield of acrylic acid may be further increased when producing acrylic acid using the catalyst for dehydration reaction.

In an embodiment of the present disclosure, calcium pyrophosphate may be β-calcium pyrophosphate (β-Capp).

Further, an embodiment of the present disclosure provides a method for producing acrylic acid, comprising the step of producing acrylic acid by performing dehydration reaction of 3-hydroxypropionic acid using the catalyst.

3-hydroxypropionic acid may be prepared by converting glucose or glycerol. For example, it can be prepared by preparing a product containing allyl alcohol from a reactant containing glycerol and carboxylic acid, injecting a heterogeneous catalyst and a basic solution into the product, and oxidizing them. A specific preparation method is as described in Patent Document KR 10-2015-0006349 A.

The step of producing acrylic acid by performing dehydration reaction of 3-hydroxypropionic acid is performed as in the following reaction formula.

[Reaction Formula]

The dehydration reaction may be carried out in any one reactor selected from the group consisting of a batch reactor, a semi-batch reactor, a continuous stirring tank reactor, a plug flow reactor, a fixed bed reactor, and a fluidized bed reactor, which are provided with the catalyst for dehydration reaction of 3-hydroxypropionic acid, or a mixed reactor to which two or more of them are connected.

The dehydration reaction may be carried out at 70 to 400 °C, preferably 100 to 300 °C, and more preferably 70 to 280 °C.

The dehydration reaction may be operated under reduced pressure, normal pressure, and pressure. Preferably, a vacuum distillation method may be used. During the vacuum distillation, the pressure may be controlled to 5 to 50 mbar, 10 to 40 mbar, or 15 to 30 mbar. If the pressure is less than 5 mbar, organic solvents other than acrylic acid are also recovered so that it may be difficult to recover high-purity acrylic acid, and if it exceeds 50 mbar, the dehydration reaction to acrylic acid may not occur, or the recovery of acrylic acid may be difficult.

[Mode for Carrying Out the Invention]

Hereinafter, the present disclosure will be described in more detail through Examples. However, the following Examples are means for explaining the present disclosure, and the right scope of the present disclosure is not limited to the matters described in Examples.

**<Preparation of Catalyst>**

## (1) Example 1

A first phosphate solution A in which 23g (=0.75M) of sodium phosphate ($Na_3PO_4 12H_2O$) had been dissolved in 80 mL of distilled water, and a calcium salt solution B (molar concentration: 1.19M) in which 14 g of a calcium chloride hydrate ($CaCl_2 2H_2O$) had been dissolved in 80 mL of distilled water were prepared.

A bath was prepared by dropping the calcium salt solution B into 40 ml of distilled water of room temperature at a rate of 3 ml/min for 30 seconds. The first phosphate solution was dropped into the bath at a rate of 3 ml/min for 30 seconds. At this time, particles may be formed under conditions in which the concentration of calcium salt in the mother liquid of the bath is high during dropping.

A $Ca_5(PO_4)_3OH$ cake was prepared by filtering, filtering, and washing a slurry formed while the solution was mixed.

A second phosphate solution C in which 23 g (=0.21 M) of sodium pyrophosphate ($Na_2P_2O_7$) had been dissolved in 180 mL of distilled water, and a calcium salt solution D (molar concentration: 1.02M) in which 27 g of a calcium chloride hydrate ($CaCl_2 2H_2O$) had been dissolved in 180 mL of distilled water were prepared.

A bath was prepared by dropping the calcium salt solution D into 40 ml of distilled water of room temperature at a rate of 3 ml/min for 30 seconds. The second phosphate solution was dropped into the bath at a rate of 3 ml/min.

A β-calcium pyrophosphate cake was prepared by filtering, filtering, and washing a slurry formed while the solution was mixed.

The prepared apatite ($Ca_5(PO_4)_3OH$) cake and β-calcium pyrophosphate cake were mixed in 1 L of water at a weight ratio of 20:80, and stirred at room temperature for about 2 hours. Thereafter, the process of filtering water by filtering the stirred solution was repeated three times. Thereafter, a cake obtained through the washing process was dried and fired at 500 °C to prepare a mixed-phase catalyst having a weight ratio of the hydroxyapatite phase to the calcium pyrophosphate phase of 20:80.

## (2) Example 2

A mixed-phase catalyst having a weight ratio of the hydroxyapatite phase and the calcium pyrophosphate phase of 70:30 was prepared in the same manner as in Example 1 except that the prepared apatite ($Ca_5(PO_4)_3OH$) cake and β-calcium pyrophosphate cake were mixed in 1L of water at a weight ratio of 70:30.

## (3) Example 3

A mixed-phase catalyst having a weight ratio of the hydroxyapatite phase to the calcium pyrophosphate phase of 15:85 was prepared in the same manner as in Example 1 except that the prepared apatite ($Ca_5(PO_4)_3OH$) cake and β-calcium pyrophosphate cake were mixed in 1L of water at a weight ratio of 15:85.

## (4) Comparative Example 1

A first phosphate solution A in which 23 g of sodium phosphate ($Na_3PO_4 12H_2O$) had been dissolved in 80 mL of distilled water and a calcium salt solution B in which 14 g of a calcium chloride hydrate ($CaCl_2 2H_2O$) had been dissolved in 80 mL of distilled water were prepared.

A bath was prepared by dropping the calcium salt solution B into 40 ml of distilled water of room temperature at a rate of 3 ml/min for 30 seconds. The first phosphate solution was dropped into the bath at a rate of 3 ml/min.

A $Ca_5(PO_4)_3OH$ cake was prepared by filtering, filtering, and washing a slurry formed while the solution was mixed.

After mixing the prepared $Ca_5(PO_4)_3OH$ cake with 1L of water, the process of filtering water by filtering the mixed solution was repeated three times. Thereafter, a cake obtained through the washing process was dried and fired at 500 °C to prepare a HAP single-phase catalyst.

## (5) Comparative Example 2

A second phosphate solution C in which 23 g (=0.21M) of sodium pyrophosphate ($Na_2P_2O_7$) had been dissolved in 180 mL of distilled water and a calcium salt solution D (molar concentration: 1.02M) in which 27 g of a calcium chloride hydrate ($CaCl_2 2H_2O$) had been dissolved in 180 mL of distilled water were prepared.

A bath was prepared by dropping the calcium salt solution D into 40 ml of distilled water of room temperature at a rate of 3 ml/min for 30 seconds. The second phosphate solution was dropped into the bath at a rate of 3 ml/min.

A β-calcium pyrophosphate cake was prepared by filtering, filtering, and washing a slurry formed while the solution was mixed.

After mixing the prepared β-calcium pyrophosphate cake with 1L of water, the process of filtering water by filtering the mixed solution was repeated three times. Thereafter, a cake obtained through the washing process was dried and fired at 500 °C to prepare a β-calcium pyrophosphate single-phase catalyst.

**(6) Comparative Example 3**

A titanium dioxide catalyst having an anatase phase ($TiO_2$, manufactured by Thermo Fisher Scientific) was prepared.

**(7) Comparative Example 4**

A silica gel catalyst (silica gel, manufactured by Aladdin) was prepared.

**<Experimental Example> Dehydration Reaction of 3-hydroxypropionic acid**

After filling 0.4 g of the catalyst prepared in Example 1 above in a quartz reaction tube having a diameter of 1/2 inch, the reaction tube was connected and fastened to a reactor furnace.

The temperature was raised to a reaction temperature of 280 °C (temperature increase rate: about 12.5 °C/min) while purging a nitrogen carrier gas ($N_2$) in the reaction tube at a rate of 25 sccm.

After reaching the reaction temperature, an aqueous 3-hydroxypropionic acid solution (concentration: 20% by weight) was injected into an upper end portion of the reactor at 1.5 mL/h using a syringe pump.

The injected 3-hydroxypropionic acid was vaporized within the reaction tube and was converted to acrylic acid through dehydration reaction in the presence of a catalyst. After that, acrylic acid condensed in a liquid phase was obtained in a sample trap (maintained at 3°C) at a lower end portion of the reaction tube (Experimental Example 1). At this time, High-performance Liquid Chromatography (HPLC) and Gas Chromatography Flame Ionization Detector (GC-FID) were used to quantify production of acrylic acid, and production and consumption amount of 3-hydroxypropionic acid in mole numbers.

At this time, the yield of acrylic acid obtained by Experimental Example 1 was 94.9%.

In addition, acrylic acid was produced by performing dehydration reaction of 3-hydroxypropionic acid in the same manner as in producing acrylic acid using the catalyst of Example 1 except that the experimental conditions of the catalysts of Examples 2 and 3 and Comparative Examples 1 to 4 prepared above such as catalyst loading amount, reaction temperature, carrier gas, and carrier gas flow amount were changed as shown in Table 1 below (Experimental Examples 2 to 10).

The yields of acrylic acid obtained by Experimental Examples 2 to 10 are as shown in Table 1 below.

At this time, the yields of acrylic acid were calculated through Equations 1 to 3 below.

3-hydroxypropionic acid conversion rate (%) = 100 × (mole number of 3-hydroxypropionic acid before reaction - mole number of 3-hydroxypropionic acid after reaction)/(mole number of 3-hydroxypropionic acid before reaction)  [Equation 1]

Acrylic acid selectivity (%) = 100 × (mole number of acrylic acid produced)/(mole number of 3-hydroxypropionic acid reacted)  [Equation 2]

Acrylic acid yield (%) = (3-hydroxypropionic acid conversion rate × acrylic acid selectivity)/100  [Equation 3]

[Table 1]

| Experimental Example | Catalyst type | Catalyst loading amount | Reaction temperature | Carrier gas | Carrier gas flow amount (sccm) | Acrylic acid yield (%) |
|---|---|---|---|---|---|---|
| Experimental Example 1 | Example 1 (HAP+β-$Ca_2P_2O_7$, 20:80) | 0.4 | 280 | $N_2$ | 25 | 94.9 |
| Experimental Example 2 | Example 1 (HAP+β-$Ca_2P_2O_7$,20:80) | 0.4 | 300 | $N_2$ | 25 | 92.5 |
| Experimental Example 3 | Example 2 (HAP+β-$Ca_2P_2O_7$,70:30) | 0.4 | 280 | $N_2$ | 25 | 87.3 |

(continued)

| Experimental Example | Catalyst type | Catalyst loading amount | Reaction temperature | Carrier gas | Carrier gas flow amount (sccm) | Acrylic acid yield (%) |
|---|---|---|---|---|---|---|
| Experimental Example 4 | Example 3 (HAP+$\beta$-Ca$_2$P$_2$O$_7$,15:85) | 0.4 | 280 | N$_2$ | 25 | 87.6 |
| Experimental Example 5 | Comparative Example 1 (HAP) | 1.6 | 280 | N$_2$ | 5 | 75.6 |
| Experimental Example 6 | Comparative Example 1 (HAP) | 1.6 | 280 | N$_2$+Ar | 50 | 62.8 |
| Experimental Example 7 | Comparative Example 2 ($\beta$-Ca$_2$P$_2$O$_7$) | 0.4 | 280 | N$_2$ | 25 | 81.2 |
| Experimental Example 8 | Comparative Example 3 (TiO$_2$) | 0.4 | 280 | N$_2$ | 25 | 77.3 |
| Experimental Example 9 | Comparative Example 4 (Silica) gel | 0.4 | 280 | N$_2$ | 25 | 73.4 |

Although the titanium dioxide and silica gel catalysts used in Experimental Examples 8 and 9 had been known to have an excellent effect on the conventional 3-hydroxypropionic acid dehydration reaction, it could be confirmed from Table 1 above that the yield of acrylic acid was less than 80%, and thus there was a limit to the yield.

Further, it could be confirmed from Table 1 above that the yields of acrylic acid were dropped in the single-phase catalysts of hydroxyapatite (HAP) used in Experimental Examples 5 and 6 even when the catalyst loading amounts were increased.

It could be confirmed from Experimental Examples 1, 3, 4, and 7 of Table 1 above that the yield of acrylic acid was dropped in the $\beta$-calcium pyrophosphate single-phase catalyst compared to those of the catalysts having a mixed phase of hydroxyapatite and calcium pyrophosphate even when acrylic acid was obtained under the same catalyst conditions.

Meanwhile, even under the same catalyst conditions, it could be confirmed that the case of using the catalysts having a mixed phase of hydroxyapatite and calcium pyrophosphate showed higher yields of acrylic acid than the case of using $\beta$-calcium pyrophosphate single-phase catalyst, or TiO$_2$ or silica gel as a catalyst, and it could be confirmed that the case of using the catalysts having a mixed phase of hydroxyapatite and calcium pyrophosphate showed high yields of acrylic acid even though the catalyst loading amounts were small compared to the case of using the single-phase catalyst of hydroxyapatite.

That is, it could be confirmed that the catalysts having the mixed phase according to the present disclosure has an effect of exhibiting a high acrylic acid yield when used in a process for producing acrylic acid by performing dehydration reaction of 3-hydroxypropionic acid.

## Claims

1.    A method for preparing the catalyst of claim 13 for dehydration reaction of 3-hydroxypropionic acid, including hydroxyapatite (HAP) and calcium pyrophosphate, the method comprising the steps of:

preparing an apatite cake by dropping a first phosphate solution into a first calcium salt solution;
preparing a calcium pyrophosphate (CaPP) cake by dropping a second phosphate solution into a second calcium salt solution;
preparing a calcium phosphate cake by mixing the apatite cake and the calcium pyrophosphate cake; and
firing the calcium phosphate cake.

2.    The method of claim 1, wherein the apatite cake and the calcium pyrophosphate cake in the step of preparing the calcium phosphate cake by mixing the apatite cake and the calcium pyrophosphate cake have a weight ratio of 10:90 to 80:20.

3.    The method of claim 1, wherein the first calcium salt solution and the second calcium salt solution each have a calcium salt concentration of 0.1M or more and 5M or less.

4. The method of claim 1, wherein the first phosphate solution and the second phosphate solution each have a phosphate concentration of 0.1M or more and 5M or less.

5. The method of claim 1, wherein the step of preparing the apatite cake by dropping the first phosphate solution into the first calcium salt solution comprises a step of dropping the first phosphate solution into a bath containing the first calcium salt solution, and the step of preparing the calcium pyrophosphate cake by dropping the second phosphate solution into the second calcium salt solution comprises a step of dropping the second phosphate solution into a bath containing the second calcium salt solution.

6. The method of claim 1, wherein the dropping of the first phosphate solution and the dropping of the second phosphate solution are each performed for 30 seconds or more and 1 hour or less.

7. The method of claim 1, wherein the first phosphate is one or more selected from the group consisting of $Li_3PO_4$, $Na_3PO_4$, and $K_3PO_4$, or hydrates thereof.

8. The method of claim 1, wherein the second phosphate is one or more selected from the group consisting of pyrophosphoric acid ($H_4P_2O_7$), $Li_4P_2O_7$, $Na_4P_2O_7$, and $K_4P_2O_7$, or hydrates thereof.

9. The method of claim 1, wherein the first calcium salt solution and the second calcium salt solution contain one or more calcium salts selected from the group consisting of calcium chloride, calcium nitrate, calcium sulfate, and calcium acetate, or hydrates thereof.

10. The method of claim 1, wherein the step of preparing the calcium phosphate cake by mixing the apatite cake and the calcium pyrophosphate cake comprises the steps of: preparing a calcium phosphate slurry by mixing the apatite cake and the calcium pyrophosphate cake; and filtering, washing, and drying the calcium phosphate slurry to prepare a calcium phosphate cake.

11. The method of claim 10, wherein the step of preparing the calcium phosphate slurry by mixing the apatite cake and the calcium pyrophosphate cake comprises a step of performing stirring at a temperature of 20 to 60 °C for 1 to 48 hours.

12. The method of claim 1, the firing is performed at a temperature condition of 300 to 700 °C.

13. A catalyst for dehydration reaction of 3-hydroxypropionic acid, obtainable by the method according to claim 1, the catalyst including a hydroxyapatite phase and a calcium pyrophosphate phase, wherein the hydroxyapatite phase and the calcium pyrophosphate phase of the catalyst have a weight ratio of 10:90 to 80:20.

14. A method for producing acrylic acid, the method comprising the step of producing acrylic acid by performing dehydration reaction of 3-hydroxypropionic acid using the catalyst according to claim 13.

15. The method of claim 14, wherein the dehydration reaction is carried out at a temperature condition of 70 to 400 °C.

**Patentansprüche**

1. Verfahren zur Herstellung des Katalysators nach Anspruch 13 für die Dehydratisierungsreaktion von 3-Hydroxypropionsäure, einschließlich Hydroxyapatit (HAP) und Calciumpyrophosphat, wobei das Verfahren die Schritte umfasst:

   Herstellen eines Apatitkuchens durch Tropfen einer ersten Phosphatlösung in eine erste Calciumsalzlösung;
   Herstellen eines Calciumpyrophosphat (CaPP)-Kuchens durch Tropfen einer zweiten Phosphatlösung in eine zweite Calciumsalzlösung;
   Herstellen eines Calciumphosphatkuchens durch Mischen des Apatitkuchens und des Calciumpyrophosphatkuchens; und
   Brennen des Calciumphosphatkuchens.

2. Verfahren nach Anspruch 1, wobei der Apatitkuchen und der Calciumpyrophosphatkuchen in dem Schritt des Herstellens des Calciumphosphatkuchens durch Mischen des Apatitkuchens und des Calciumpyrophosphatkuchens ein Gewichtsverhältnis von 10:90 bis 80:20 aufweisen.

3.  Verfahren nach Anspruch 1, wobei die erste Calciumsalzlösung und die zweite Calciumsalzlösung jeweils eine Calciumsalzkonzentration von 0,1 M oder mehr und 5 M oder weniger aufweisen.

4.  Verfahren nach Anspruch 1, wobei die erste Phosphatlösung und die zweite Phosphatlösung jeweils eine Phosphatkonzentration von 0,1 M oder mehr und 5 M oder weniger aufweisen.

5.  Verfahren nach Anspruch 1, wobei der Schritt des Herstellens des Apatitkuchens durch Tropfen der ersten Phosphatlösung in die erste Calciumsalzlösung einen Schritt des Tropfens der ersten Phosphatlösung in ein Bad umfasst, das die erste Calciumsalzlösung enthält, und der Schritt des Herstellens des Calciumpyrophosphatkuchens durch Tropfen der zweiten Phosphatlösung in die zweite Calciumsalzlösung einen Schritt des Tropfens der zweiten Phosphatlösung in ein Bad umfasst, das die zweite Calciumsalzlösung enthält.

6.  Verfahren nach Anspruch 1, wobei das Tropfen der ersten Phosphatlösung und das Tropfen der zweiten Phosphatlösung jeweils für 30 Sekunden oder mehr und 1 Stunde oder weniger durchgeführt werden.

7.  Verfahren nach Anspruch 1, wobei das erste Phosphat eines oder mehrere ausgewählt aus der Gruppe bestehend aus $Li_3PO_4$, $Na_3PO_4$ und $K_3PO_4$ oder Hydraten davon ist.

8.  Verfahren nach Anspruch 1, wobei das zweite Phosphat eines oder mehrere ausgewählt aus der Gruppe bestehend aus Pyrophosphorsäure ($H_4P_2O_7$), $Li_4P_2O_7$, $Na_4P_2O_7$ und $K_4P_2O_7$ oder Hydraten davon ist.

9.  Verfahren nach Anspruch 1, wobei die erste Calciumsalzlösung und die zweite Calciumsalzlösung ein oder mehrere Calciumsalze ausgewählt aus der Gruppe bestehend aus Calciumchlorid, Calciumnitrat, Calciumsulfat und Calciumacetat oder Hydraten davon enthalten.

10. Verfahren nach Anspruch 1, wobei der Schritt des Herstellens des Calciumphosphatkuchens durch Mischen des Apatitkuchens und des Calciumpyrophosphatkuchens die Schritte umfasst: Herstellen einer Calciumphosphataufschlämmung durch Mischen des Apatitkuchens und des Calciumpyrophosphatkuchens; und Filtern, Waschen und Trocknen der Calciumphosphataufschlämmung, um einen Calciumphosphatkuchen herzustellen.

11. Verfahren nach Anspruch 10, wobei der Schritt des Herstellens der Calciumphosphataufschlämmung durch Mischen des Apatitkuchens und des Calciumpyrophosphatkuchens einen Schritt des Durchführens von Rühren bei einer Temperatur von 20 bis 60 °C für 1 bis 48 Stunden umfasst.

12. Verfahren nach Anspruch 1, wobei das Brennen bei einer Temperaturbedingung von 300 bis 700 °C durchgeführt wird.

13. Katalysator für die Dehydratisierungsreaktion von 3-Hydroxypropionsäure, erhältlich durch das Verfahren nach Anspruch 1, wobei der Katalysator eine Hydroxyapatitphase und eine Calciumpyrophosphatphase einschließt, wobei die Hydroxyapatitphase und die Calciumpyrophosphatphase des Katalysators ein Gewichtsverhältnis von 10:90 bis 80:20 aufweisen.

14. Verfahren zur Herstellung von Acrylsäure, wobei das Verfahren den Schritt des Herstellens von Acrylsäure durch Durchführen einer Dehydratisierungsreaktion von 3-Hydroxypropionsäure unter Verwendung des Katalysators nach Anspruch 13 umfasst.

15. Verfahren nach Anspruch 14, wobei die Dehydratisierungsreaktion bei einer Temperaturbedingung von 70 bis 400 °C durchgeführt wird.

**Revendications**

1.  Procédé de préparation du catalyseur selon la revendication 13 pour une réaction de déshydratation d'acide 3-hydroxypropionique, incluant une hydroxyapatite (HAP) et un pyrophosphate de calcium, le procédé comprenant les étapes consistant à :

    préparer un gâteau d'apatite en déposant une première solution de phosphate dans une première solution de sel de calcium ;

préparer un gâteau de pyrophosphate de calcium (CaPP) en déposant une deuxième solution de phosphate dans une deuxième solution de sel de calcium ;

préparer un gâteau de phosphate de calcium en mélangeant le gâteau d'apatite et le gâteau de pyrophosphate de calcium ; et

cuire le gâteau de phosphate de calcium.

2. Procédé selon la revendication 1, dans lequel le gâteau d'apatite et le gâteau de pyrophosphate de calcium de l'étape de préparation du gâteau de phosphate de calcium en mélangeant le gâteau d'apatite et du gâteau de pyrophosphate de calcium ont un rapport en poids de 10:90 à 80:20.

3. Procédé selon la revendication 1, dans lequel la première solution de sel de calcium et la deuxième solution de sel de calcium ont chacune une concentration de sel de calcium de 0,1 M ou plus et 5 M ou moins.

4. Procédé selon la revendication 1, dans lequel la première solution de phosphate et la deuxième solution de phosphate ont chacune une concentration de phosphate de 0,1 M ou plus et de 5 M ou moins.

5. Procédé selon la revendication 1, dans lequel l'étape de préparation du gâteau d'apatite en déposant la première solution de phosphate dans la première solution de sel de calcium comprend une étape de dépôt de la première solution de phosphate dans un bain contenant la première solution de sel de calcium, et l'étape de préparation du gâteau de pyrophosphate de calcium en déposant la deuxième solution de phosphate dans la deuxième solution de sel de calcium comprend une étape de dépôt de la deuxième solution de phosphate dans un bain contenant la deuxième solution de sel de calcium.

6. Procédé selon la revendication 1, dans lequel le dépôt de la première solution de phosphate et le dépôt de la deuxième solution de phosphate sont chacun effectués pendant 30 secondes ou plus et 1 heure ou moins.

7. Procédé selon la revendication 1, dans lequel le premier phosphate est un ou plusieurs éléments sélectionnés dans le groupe constitué de $Li_3PO_4$, $Na_3PO_4$ et $K_3PO_4$, ou des hydrates de ces derniers.

8. Procédé selon la revendication 1, dans lequel le deuxième phosphate est un ou plusieurs éléments sélectionnés dans le groupe constitué d'acide pyrophosphorique ($H_4P_2O_7$), $Li_4P_2O_7$, $Na_4P_2O_7$ et $K_4P_2O_7$, ou des hydrates de ces derniers.

9. Procédé selon la revendication 1, dans lequel la première solution de sel de calcium et la deuxième solution de sel de calcium contiennent un ou plusieurs sels de calcium sélectionnés dans le groupe constitué de chlorure de calcium, nitrate de calcium, sulfate de calcium et acétate de calcium, ou des hydrates de ces derniers.

10. Procédé selon la revendication 1, dans lequel l'étape de préparation du gâteau de phosphate de calcium en mélangeant le gâteau d'apatite et le gâteau de pyrophosphate de calcium comprend les étapes consistant à : préparer une suspension de phosphate de calcium en mélangeant le gâteau d'apatite et le gâteau de pyrophosphate de calcium ; et filtrer, laver et sécher la suspension de phosphate de calcium pour préparer un gâteau de phosphate de calcium.

11. Procédé selon la revendication 10, dans lequel l'étape de préparation de la suspension de phosphate de calcium en mélangeant le gâteau d'apatite et le gâteau de pyrophosphate de calcium comprend une étape consistant à effectuer l'agitation à une température de 20 à 60°C pendant 1 à 48 heures.

12. Procédé selon la revendication 1, dans lequel la cuisson est effectuée à une température de 300 à 700°C.

13. Catalyseur de réaction de déshydratation d'acide 3-hydroxypropionique, obtenable par le procédé selon la revendication 1, le catalyseur incluant une phase hydroxyapatite et une phase pyrophosphate de calcium, dans lequel la phase hydroxyapatite et la phase pyrophosphate de calcium du catalyseur ont un rapport en poids de 10:90 à 80:20.

14. Procédé de production d'acide acrylique, le procédé comprenant l'étape de production de l'acide acrylique en effectuant une réaction de déshydratation d'acide 3-hydroxypropionique en utilisant le catalyseur selon la revendication 13.

15. Procédé selon la revendication 14, dans lequel la réaction de déshydratation est effectuée à une température de 70 à

400°C.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020120025888 A
- KR 20120025888 A

- KR 1020150006349 A